(19) Europäisches Patentamt
European Patent Office
Office européen des brevets

(11) Veröffentlichungsnummer: **0 432 595 A1**

(12) # EUROPÄISCHE PATENTANMELDUNG

(21) Anmeldenummer: **90123033.4**

(51) Int. Cl.5 **C07D 213/40**, A61K 31/44

(22) Anmeldetag: **01.12.90**

(30) Priorität: **14.12.89 DE 3941235**

(43) Veröffentlichungstag der Anmeldung:
**19.06.91 Patentblatt 91/25**

(84) Benannte Vertragsstaaten:
**AT BE CH DE DK ES FR GB GR IT LI LU NL SE**

(71) Anmelder: **BAYER AG**
**W-5090 Leverkusen 1 Bayerwerk(DE)**

(72) Erfinder: **Voges, Klaus-Peter, Dr.**
**Schmitteborn 161**
**W-5600 Wuppertal 22(DE)**
Erfinder: **Häbich, Dieter, Dr.**
**Krummacherstrasse 82**
**W-5600 Wuppertal 1(DE)**
Erfinder: **Hansen, Jutta, Dr.**
**Claudiusweg 9**
**W-5600 Wuppertal 1(DE)**
Erfinder: **Paessens, Arnold, Dr.**
**Stresemannstrasse 51**
**W-5657 Haan(DE)**
Erfinder: **Meichsner, Christoph, Dr.**
**Biener Strasse 30**
**W-6238 Hofheim 1(DE)**

(54) **Neue Peptide, ihre Herstellung und ihre Verwendung in Arzneimitteln.**

(57) Die Erfindung betrifft neue Peptide der allgemeinen Formel (I)

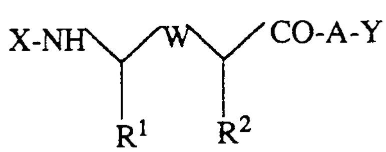

(I)

in der die Substituenten die in der Beschreibung angegebene Bedeutung haben, Verfahren zu ihrer Herstellung und ihre Verwendung als Arzneimittel, insbesondere als antivirale Mittel in der Human- und Tiermedizin.

EP 0 432 595 A1

## NEUE PEPTIDE, IHRE HERSTELLUNG UND IHRE VERWENDUNG IN ARZNEIMITTELN

Die Erfindung betrifft neue Peptide, Verfahren zu ihrer Herstellung und ihre Verwendung als Arzneimittel, insbesondere als antivirale Mittel in der Human- und Tiermedizin.

In den GB 2 203 740 und EP-A-2 0 337 714 werden Peptide beschrieben, die antivirale Aktivität gegen das "human immunodeficiency virus" (HIV) besitzen.

Die vorliegende Erfindung stellt nun Peptide der allgemeinen Formel (I),

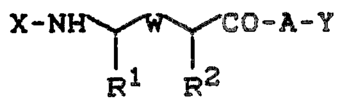

(I)

zur Verfügung,
in welcher
X - für eine Gruppe der Formel $R^3CO$- steht,
worin
$R^3$ - geradkettiges oder verzweigtes Alkyl mit bis zu 8 Kohlenstoffatomen bedeutet oder
- Aryl mit 6 bis 10 Kohlenstoffatomen oder Tolyl bedeutet

$R^1$ - für Cyclohexylmethyl steht,

$R^2$ - für Isopropyl steht,

W - für einen Rest der Formel

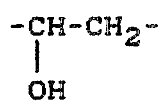

steht,

A - für einen Rest der Formel

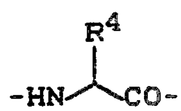

steht

worin
$R^4$ - geradkettiges oder verzweigtes Alkyl mit bis zu 8 Kohlenstoffatomen bedeutet
in der D- oder L-Form, oder als D,L-Isomerengemisch, bevorzugt in der L-Form,
Y - für eine Gruppe der Formel $-NHR^5$ steht,
worin
$R^5$ - die Gruppe

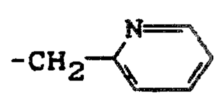

bedeutet

und ihre physiologisch unbedenklichen Salze.

Die allgemeine Formel Ia

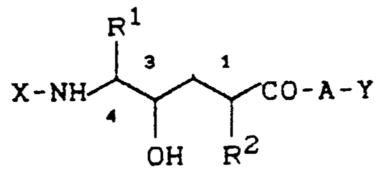 (Ia)

besitzt 3 asymmetrische Kohlenstoffatome (1, 3 und 4), die unabhängig voneinander in der R- oder S-Konfiguration vorliegen können. Bevorzugt liegt diese Gruppe in der 1R, 3S, 4S-Konfiguration , 1R, 3R, 4S-Konfiguration, 1S, 3R, 4S-Konfiguration oder in der 1S, 3S, 4S-Konfiguration vor. Besonders bevorzugt ist die 1S, 3S, 4S-Konfiguration.

Die erfindungsgemäßen Verbindungen der allgemeinen Formel (I) können in Form ihrer Salze vorliegen. Dies können Salze mit anorganischen oder organischen Säuren oder Basen sein. Zu den Säureadditionsprodukten gehören bevorzugt Salze mit Salzsäure, Bromwasserstoffsäure, Iodwasserstoffsäure, Schwefelsäure, Phosphorsäure oder mit Carbonsäuren wie Essigsäure, Propionsäure, Oxalsäure, Glykolsäure, Bernsteinsäure, Maleinsäure, Hydroxymaleinsäure, Methylmaleinsäure, Fumarsäure, Adipinsäure, Äpfelsäure, Weinsäure, Zitronensäure, Benzoesäure, Zimtsäure, Milchsäure, Ascorbinsäure, Salicylsäure, 2-Acetoxybenzoesäure, Nicotinsäure, Isonicotinsäure, oder Sulfonsäuren wie Methansulfonsäure, Ethansulfonsäure, Benzolsulfonsäure, Toluolsulfonsäure, Naphthalin-2-sulfonsäure oder Naphthalindisulfonsäure.

Bevorzugt sind Verbindungen der allgemeinen Formel (I)
in welcher
X - für eine Gruppe der Formel $R^3CO$- steht
worin
$R^3$ - geradkettiges oder verzweigtes Alkyl mit bis zu 6 Kohlenstoffatomen, Phenyl oder Tolyl bedeutet

$R^1$ - für Cyclohexylmethyl steht,

$R^2$ - für Isopropyl steht,

W - für einen Rest der Formel

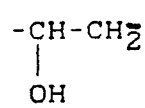 steht

A- für einen Rest der Formel

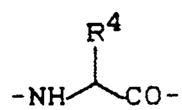 steht

worin
$R^4$ - geradkettiges oder verzweigtes Alkyl mit bis zu 6 Kohlenstoffatomen bedeutet

in der D- oder L-Form, oder als D,L-Isomerengemisch, bevorzugt in der L-Form
Y - für eine Gruppe der Formel $NHR^5$ steht, worin
$R^5$ die Gruppe

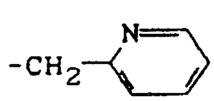 bedeutet

und ihre physiologisch unbedenklichen Salze.

Besonders bevorzugt sind Verbindungen der allgemeinen Formel (I)

in welcher
X - für eine Gruppe der Formel $R^3CO$- steht
worin
$R^3$ - geradkettiges oder verzweigtes Alkyl mit bis zu 4 Kohlenstoffatomen, Tolyl oder Phenyl bedeutet

$R^1$ - für Cyclohexylmethyl steht,

$R^2$ - für Isopropyl steht,

W - für einen Rest der Formel

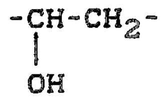 steht,

A - für einen Rest der Formel

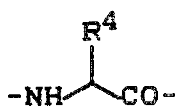 steht

worin
$R^4$ - geradkettiges oder verzweigtes Alkyl mit bis zu 4 Kohlenstoffatomen bedeutet
in der D- oder L-Form, oder als D,L-Isomerengemisch, bevorzugt in der L-Form
Y - für eine Gruppe der Formel $NHR^5$ steht, worin
$R^5$ die Gruppe

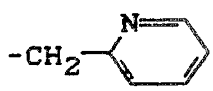 bedeutet

und ihre physiologisch unbedenklichen Salze.

Außerdem wurde ein Verfahren zur Herstellung der Verbindungen dar allgemeinen Formel (I) gefunden, das dadurch gekennzeichnet ist, daß man die
Verbindungen der allgemeinen Formel (II)

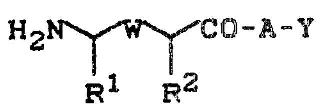 (II)

in welcher
$R^1$, $R^2$, W, A und Y die oben angegebene Bedeutung haben,
nach üblicher Methode, gegebenenfalls in Anwesenheit einer Base, in inerten Lösemitteln acyliert.

Das erfindungsgemäße Verfahren kann durch folgendes Formelschema beispielhaft erläutert werden:

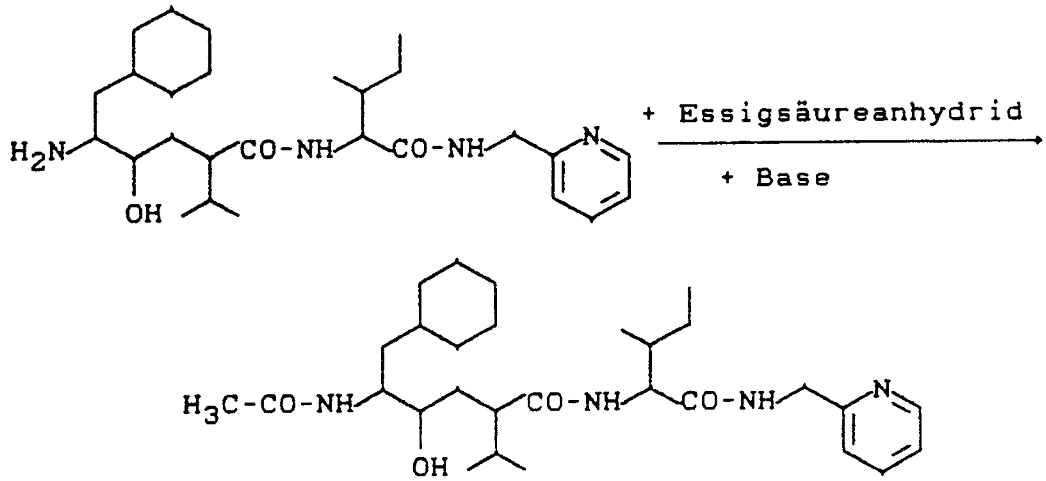


Als Lösemittel für die Acylierung eignen sich die üblichen inerten Lösemittel, die sich unter den Reaktionsbedingungen nicht verändern. Hierzu gehören bevorzugt organische Lösemittel wie Ether z.B. Diethylether, Glykolmono-oder dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenwasserstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt ist Dimethylformamid.

Als Acyclierungsmittel eignen sich beispielsweise Carbonsäureanhydride, Carbonsäurechloride oder Carbonsäureester. Bevorzugt sind Carbonsäureanhydride wie beispielsweise Essigsäureanhydrid.

Als Basen eignen sich die üblichen organischen Basen. Hierzu gehören bevorzugt organische Amine wie Triethylamin, Picolin oder N-Methylpiperidin. Besonders bevorzugt ist N-Methylpiperidin.

Die Acylierung wird im allgemeinen in einem Temperaturbereich von 0°C bis +80°C, vorzugsweise bei Raumtemperatur durchgeführt.

Die Base wird hierbei in einer Menge von 1,0 bis 1000, bevorzugt von 1,0 bis 2,0 Mol, bezogen auf 1 Mol der Verbindung der allgemeinen Formel (II) eingesetzt.

Die Verbindungen der allgemeinen Formel (II) sind an sich bekannt oder können ausgehend von entsprechenden Carbonsäureestern ($H_2N-CHR^1-W-CHR^2-COOH$) durch Umsetzung mit dem Aminosäurerest (HO-A-Y) nach den in der Peptidchemie üblichen Methoden hergestellt werden ($R^1$, $R^2$, W, A und Y haben die oben angegebene Bedeutung).

Die Verbindungen der allgemeinen Formel (I) besitzen eine außerordentlich starke Wirkung gegen Retroviren.

Dies konnte durch Versuche im HIV-spezifischen Protease-Enzymtest belegt werden.

Das Ergebnis des unten aufgeführten Beispiels wurde nach dem in der folgenden Literaturangabe [vgl. Hansen, J., Billich, S., Schulze, T. Sukrow, S. and Mölling, K. (1988), EMBO Journal, Vol. 7, No. 6, pp. 1785-1791]; beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Der angegebene $IC_{50}$-Wert bezieht sich auf die Substanzkonzentration, die unter den oben aufgeführten Testbedingungen eine 50%ige Hemmung der Protease-Aktivität bewirkt.

| Beispiel-Nr. | $IC_{50}$ (RP-HPLC) |
|---|---|
| I | $5 \times 10^{-10}$ |

Außerdem zeigen die erfindungsgemäßen Verbindungen überraschenderweise Wirkung in der infizierten Zellkultur. Dies wurde am Beispiel des Visnavirus gezeigt.

Das Visna-Virus und das HIV-Virus gehören beide zu der Retrovirus-Subfamilie der Lentiviren. Beide

Viren weisen eine ähnliche Genomorganisation und ein gegenüber den anderen Retroviren komplexes Transkriptionsmuster auf.

Bekannte Inhibitoren des HIV hemmen auch das Visnavirus in vitro in vergleichbaren Konzentrationen; d.h., dieses Modell eignet sich für die Prüfung und Auffindung von Hemmstoffen des HIV.

In Zellkulturen, die mit Visna-Virus infiziert sind, treten 5 bis 10 Tage nach der Infektion ausgeprägte virusinduzierte, zytopathische Effekte auf. Durch Behandlung der infizierten Zellkulturen mit erfindungsgemäßen Verbindungen konnte das Auftreten dieser zytopathischen Effekte verhindert werden.

Der Visna-Virus-Test wurde nach der Methode von O. Narayan et al., Journal of Infectious Diseases 135, 5, 1977, 800 - 806 durchgeführt. Dazu wurde eine erfindungsgemäße Verbindung im Kulturmedium in nicht zytotoxischen Konzentrationen in 96er Microtiterplatten verdünnt. Anschließend wurden Fibroblastenzellen vom Schaf (5 x $10^4$ Zellen pro Napf) in Produktionsmedium zu jedem Näpfchen zugeführt. Jedes Näpfchen erhielt dann 50 $\mu$l einer Visna-Viruslösung mit einer Titer von ca. 2,5 x $10^4$ $TCID_{50}$ (TCID = tissue culture infections dosis). Diese Virusdosis entspricht einer MOI (Multiplizität der Infektion) von ca. 0,05.

Unter diesen Infektionsbedingungen resultierte zwischen Tag 5 und Tag 10 in einer Infektionskontrolle ohne Substanz ein virusinduzierter, zytopathischer Effekt. Die infizierten und behandelten Zellen und die Kontrollzellen wurden 7 Tage bei 37° C 5% $CO_2$ inkubiert.

Bei Auftreten des virusinduzierten zytopathogenen Effektes in der unbehandelten Viruskontrolle wurden die Kulturen mit Formalin fixiert und anschließend mit einer Giemsa-Lösung gefärbt. Die inhibitorische Konzentration ($IC_{50}$) wurde mikroskopisch als die Konzentration ermittelt, bei der der zytopathische Effekt um 50% gehemmt wurde: im Vergleich zur unbehandelten Viruskontrolle, die 100%. Zellzerstörung aufwies.

Es wurde gefunden, daß beispielsweise die Verbindung aus Beispiel I die mit Visna-Virus infizierten Zellen vor der virusinduzierten Zellzerstörung schützte.

Die erfindungsgemäßen Verbindungen stellen somit wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung oder Prophylaxe von menschlichen Retrovirusinfektionen.

2.) Die Behandlung oder Prophylaxe von HIV I (Virus der humanen Immundefizienz; früher HTLV III/LAV genannt) und HIV II verursachten Erkrankungen (AIDS) und den damit assozierten Stadien wie ARC (AIDS related complex) und LAS (Lymphadenopathie-Syndrom) sowie der durch diese Viren verursachten Immunschwäche und Encephalopathie.

3.) Die Behandlung oder die Prophylaxe einer HTLV I-oder HTLV II-Infektion.

4.) Die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen verursacht durch das Katzenleukämievirus
g) Infektionen verursacht durch das Virus der Katzen-Immundefizienz

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den pharmazeutischen Zubereitungen im allgemeinen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Anlage zum experimentellen Teil

Liste der Dünnschichtsysteme
Ia) $CH_2Cl_2/CH_3OH$ = 90:1
Ib) $CH_2Cl_2/CH_3OH$ = 85:15

Ausgangsverbindung

Beispiel 1

$N_\alpha$-{1-[5-S-Amino-cyclohexyl-4(S)-hydroxy-2-(1-methyl)ethylhexanoyl]}-S-isoleucinyl-2-pyridylmethylamid

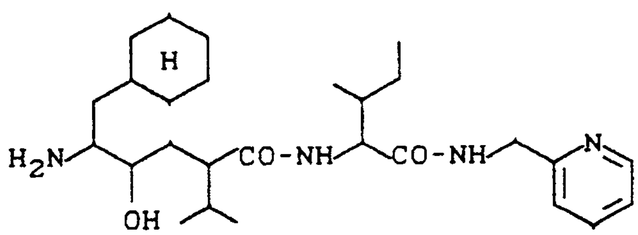

7 g (10,8 mmol) 3-Benzyloxycarbonyl-4(S)-cyclohexylmethyl-2,2-dimethyl-5(S)-{1'-{3'-[2'-(1-methyl)-ethyl]propanoyl}-S-isoleucinyl-2-pyridylmethylamid}oxazolidin werden in 120 ml Methanol und 10 ml Eisessig gelöst und mit 200 mg Palladium auf Aktivkohle versetzt. Für 10 h wird ein Strom $H_2$ durchgeleitet, danach wird die Lösung über Kieselgur abgesaugt und mit 50 ml Kaliumhydrogensulfat-Lösung versetzt. Nach Abdestillieren des Methanols wird mit 80 ml Wasser verdünnt und zweimal mit Ethylacetat extrahiert. Die wässrige Lösung wird mit 2N NaOH auf pH 10 gebracht und dreimal mit Ethylacetat extrahiert Nach Trocknen und Einengen der organischen Phase erhält man 3,2 g eines wachsartigen Feststoffs
DG: $R_f$ (Ia) = 0,80
MS (FAB) = 475
SF, MG: $C_{27}H_{46}N_4O_3$ (474,3)

Herstellungsbeispiel (allgemeine Formel I)

Beispiel I

$N\alpha$-[$N\delta$-[1-(1-Oxo)ethyl-[1-(5-S-amino-6-cyclohexyl-4-S-hydroxy-2-(1-methyl)ethylhexanoyl)]]-S-isoleucinyl}-2-pyridylmethylamid

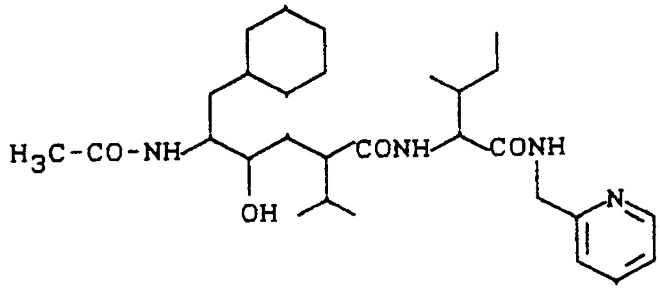

474 mg (1 mmol) der Verbindung aus Beispiel 1 werden in 4 ml Dimethylformamid gelöst und mit 113 µl Essigsäureanhydrid sowie 146 µl N-Methylpiperidin versetzt. Die Lösung wird 4 h gerührt und eingeengt.

Der Rückstand wird zwischen Ethylacetat und gesättigter Natriumhydrogencarbonatlösung verteilt. Die organische Phase wird mit Kaliumhydrogensulfatlösung (0,5 m) gewaschen, zweimal mit Wasser neutral gewaschen, getrocknet und eingeengt. Beim Einengen kristallisiert ein weißer Festkörper aus.
Ausbeute: 413 mg (80% der Theorie)
DC: $R_f$ (Ib) = 0,69
MS (FAB) = 517
SF, MG: $C_{29}H_{48}N_4O_4$ (516,73)

Beispiel II
Nα-{Nδ-[1-(1-Butanoyl)-[1-(5-8-amino-6-cyclohexyl-4-S-hydroxy-2-(1-methyl)ethylhexanoyl)]]-S-isoleucinyl}-2-pyridylmethylamid

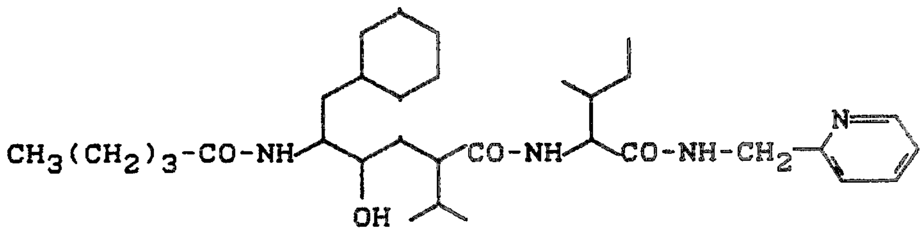

Beispiel III
Nα-{Nδ-[1-(3-Methyl)butanoyl-[1-(5-S-amino-6-cyclohexyl-4-S-hydroxy-2-(1-methyl)ethylhexanoyl)]]-S-isoleucinyl}-2-pyridylmethylamid

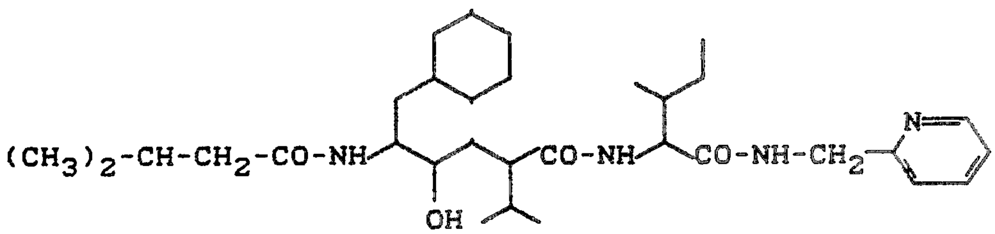

Beispiel IV
Nα-{Nδ-[1-(Benzoyl)-[1-(5-S-amino-6-cyclohexyl-4-S-hydroxy-2-(1-methyl)ethylhexanoyl)]]-S-isoleucinyl}-2-pyridylmethylamid

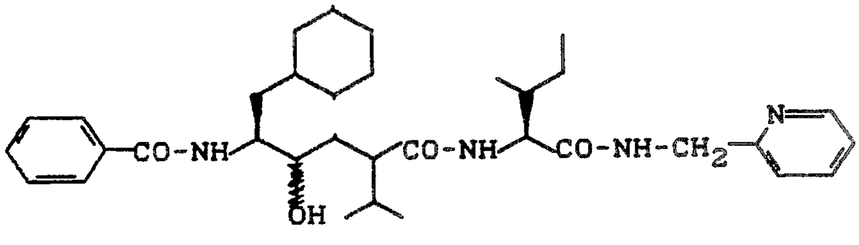

Zu einer gerührten, auf 0° C gekühlten Lösung von 240 mg (0.44 mmol) der Verbindung aus Beispiel 1 in und 0.192 ml (1.75 mmol) N-Methylmorpholin in 1.5 ml wassefreiem DMF werden 64 µl (0.55 mmol) Benzoylchlorid getropft. Nach 45 Minuten bei 0° C wird in ein Gemisch aus 20 ml kalter Natriumbicarbonatlösung und 10 ml Ethylacetat gegossen und gut durhgeführt.

Die organische Phase wird abgetrennt, die Wasserphase mit 10 ml Ethylacetat extrahiert und die vereinigten Extrakte werden über Magnesiumsulfat getrocknet. Nach Abdampfen des Lösemittels im Vakuum und Chromatographie des Rohprodukts an 20 g Kieselgel (Ethylacetat) erhält man 32 mg (13 %)

des unpolaren Isomers, Rf = 0.28 (Ethylacetat) MS (FAB) m/z = 579 $(M+H)^+$, 601 $(M+Na)^+$.
SF (MG): $C_{34}H_{50}N_4O_4$ (578.81) und 62 mg (24 %) des polaren Isomers, Rf = 0,19 (Ethylacetat) MS (FAB) m/z = 579 $(M+H)^+$, 601 $(M+Na)^+$.
SF (MG): $C_{34}H_{50}N_4O_4$ (578.81).

In Analogie zur Vorschrift des Beispiels IV erhielt man aus 100 mg (0.18 mmol) der Verbindung aus Beispiel 1 und 35 µl (0.23 mmol) l-Naphthoylchlorid nach Chromatographie des Rohprodukts an 15 g Kieselgel (Ethylacetat) 52 mg (46 %) der Titelverbindung als farbloses Pulver.
DC: Rf = 0.15 (Ethylacetat)
MS (FAB) m/z = 629 $(M+H)^+$
SF (MG): $C_{38}H_{52}N_4O_4$ (628.87).

Beispiel V
Nα-{Nδ-[1-(4-Methyl)benzoyl-[1-(5-S-amino-6-cyclohexyl-4-S-hydroxy-2-(1-methyl)ethylhexanoyl)]]-S-isoleucinyl}-2-pyridylmethylamid

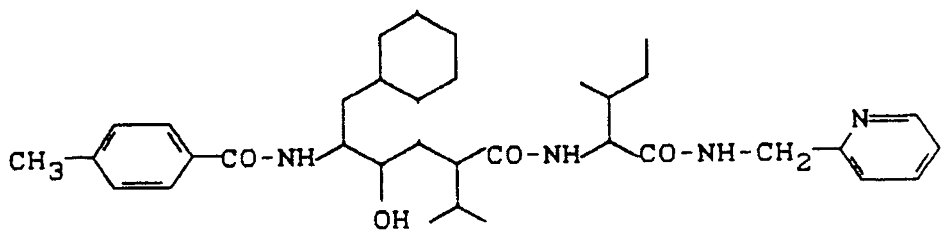

Beispiel VI
Nα-{Nδ-[1-(1-Naphthoyl)-[1-(5-S-amino-6-cyclohexyl-4-S-hydroxy-2-(1-methyl)ethylhexanoyl)]]-S-isoleucinyl}2-pyridylmethylamid

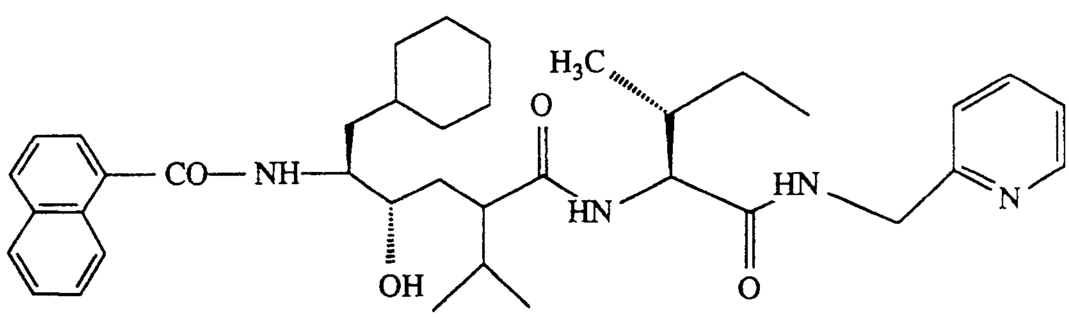

**Ansprüche**

1. Peptide der allgemeinen Formel (I),

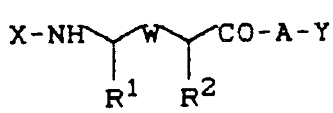

(I)

in welcher
X - für eine Gruppe der Formel $R^3CO$- steht,
worin
$R^3$ - geradkettiges oder verzweigtes Alkyl mit bis zu 8 Kohlenstoffatomen bedeutet oder

- Aryl mit 6 bis 10 Kohlenstoffatomen oder Tolyl bedeutet

$R^1$ - für Cyclohexylmethyl steht,

$R^2$ - für Isopropyl steht,

W - für einen Rest der Formel

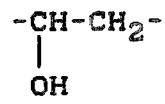 steht,

A - für einen Rest der Formel

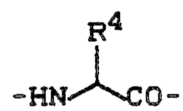

worin
$R^4$ - geradkettiges oder verzweigtes Alkyl mit bis zu 8 Kohlenstoffatomen bedeutet.
in der D- oder L-Form, oder als D,L-Isomerengemisch, bevorzugt in der L-Form
Y - für eine Gruppe der Formel $NHR^5$ steht, worin
$R^5$ die Gruppe

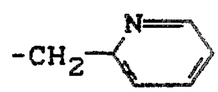 bedeutet

und ihre physiologisch unbedenklichen Salze.

2. Verbindungen nach Anspruch 1
in welcher
X - für eine Gruppe der Formel $R^3CO$- steht
worin
$R^3$ - geradkettiges oder verzweigtes Alkyl mit bis zu 6 Kohlenstoffatomen, Tolyl oder Phenyl bedeutet

$R^1$ - für Cyclohexylmethyl steht,

$R^2$ - für Isopropyl steht,

W - für einen Rest der Formel

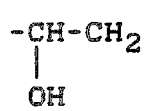 steht

A- für einen Rest der Formel

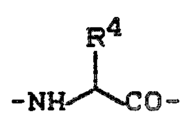 steht

worin
$R^4$ - geradkettiges oder verzweigtes Alkyl mit bis zu 6 Kohlenstoffatomen bedeutet in der D- oder L-Form, oder als D,L-Isomerengemisch, bevorzugt in der L-Form
Y - für eine Gruppe der Formel $NHR^5$ steht, worin
$R^5$ die Gruppe

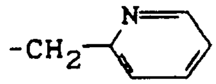 bedeutet

und ihre physiologisch unbedenklichen Salze.

3. Verbindungen nach Anspruch 1
in welcher
X - für eine Gruppe der Formel $R^3CO$- steht
worin
$R^3$ - geradkettiges oder verzweigtes Alkyl mit bis zu 4 Kohlenstoffatomen, Phenyl oder Tolyl bedeutet

$R^1$ - für Cyclohexylmethyl steht,

$R^2$ - für Isopropyl steht,

W - für einen Rest der Formel

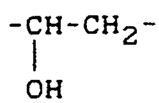 steht,

A - für einen Rest der Formel

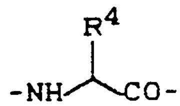 steht

worin
$R^4$ - geradkettiges oder verzweigtes Alkyl mit bis zu 4 Kohlenstoffatomen bedeutet in der D- oder L-Form, oder als D,L-Isomerengemisch, bevorzugt in der L-Form
Y - für eine Gruppe der Formel $-NHR^5$ steht,
worin
$R^5$ - die Gruppe

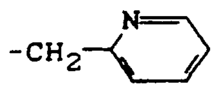 bedeutet

und ihre physiologisch unbedenklichen Salze.

4. Verbindungen nach Anspruch 1 bei denen der Rest A in der L-Form vorliegt.

5. Verbindungen gemäß Ansprüchen 1 und 2 in denen die in der allgemeinen Formel Ia

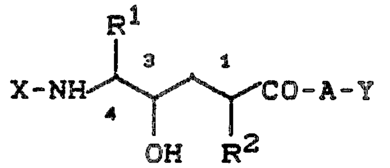

(Ia)

mit 1, 3 und 4 bezeichneten Kohlenstoffatomen in der 1R, 3S, 4S-Konfiguration , 1R, 3R, 4S-Konfiguration, 1S, 3R, 4S-Konfiguration oder in der 1S, 3S, 4S-Konfiguration vorliegen.

**6.** Verfahren zur Herstellung der Verbindungen gemäß den Ansprüchen 1-3, dadurch gekennzeichnet, daß man Verbindungen der Formel II

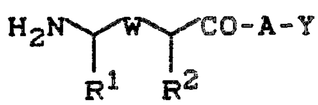

(II)

in welcher
$R^1$, $R^2$, W, A und Y die oben angegebene Bedeutung haben,
nach üblicher Methode, gegebenenfalls in Anwesenheit einer Base, in inerten Lösemitteln acyliert.

**7.** Arzneimittel enthaltend mindestens eine Verbindung der allgemeinen Formel I gemäß Anspruch 1.

**8.** Verwendung der Verbindungen der allgemeinen Formel I gemäß Anspruch 1 bei der Bekämpfung von Krankheiten.

Europäisches Patentamt

# EUROPÄISCHER RECHERCHENBERICHT

Nummer der Anmeldung

**EP 90 12 3033**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | WO-A-8 904 833 (UPJOHN)<br>* Ansprüche 1,4; Seite 1, Zeilen 5-11; Seite 43, Formel 2; Seite 45, Formel *<br>– – – | 1,7 | C 07 D 213/40<br>A 61 K 31/44 |
| D,Y | GB-A-2 203 740 (SANDOZ)<br>* Anspruch 1; Seite 17, Zeilen 16-29; Seite 18, Zeilen 1-2 *<br>– – – | 1,7 | |
| D,A | EP-A-0 337 714 (MERCK)<br>* Seiten 17, Zeile 2 *<br>– – – | 1,7 | |
| P,A | EP-A-0 357 332 (MERCK)<br>* Seite 2, Zeilen 10-30; Seite 13; Zusammenfassung *<br>– – – | 1,7 | |
| P,X | SCIENCE, Band 247, Nr. 4941, Januar 1990, Seiten 454-456, Washington, DC, US; T.J. McQUADE et al.: "A synthetic HIV-1 protease inhibitor with antiviral activity arrests HIV-like particle maturation"<br>* Seiten 454-456; Seite 454, Figur 1, Zusammenfassung *<br>– – – – – | | RECHERCHIERTE SACHGEBIETE (Int. Cl.5)<br>C 07 D 213/00<br>A 61 K 31/00 |

Der vorliegende Recherchenbericht wurde für alle Patentansprüche erstellt

| Recherchenort | Abschlußdatum der Recherche | Prüfer |
|---|---|---|
| Den Haag | 11 März 91 | ENGEL H.S.L. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument